# EUROPEAN PATENT APPLICATION

(11) **EP 4 257 094 A1**
(43) Date of publication of application: **11.10.2023**
(21) Application number: 21899761.7
(22) Date of filing: 21.10.2021
(51) Int. Cl.: A61F 2/89, A61F 2/90

(54) **MEMBRANE-COVERED STENT**

(30) Priority: 02.12.2020 CN 202011397340
(71) Applicant: Lifetech Scientific (Shenzhen) Co., Ltd., Shenzhen, Guangdong 518063 (CN)
(72) Inventor: XIAO, Benhao, Shenzhen, Guangdong 518063 (CN)
(74) Representative: Prinz & Partner mbB
(86) International application number: PCT/CN2021/125251
(87) International publication number: WO 2022/116727

(57) **Abstract**

A membrane-covered stent (100), including a first stent (110), a second stent (120) and a covering membrane (130); the covering membrane (130) is covered on the first stent (110) so as to form a lumen structure having a recess (140) in a middle portion; the second stent (120) is at least partially accommodated in the recess (140); and a longitudinal central axis (I-I) of the first stent (110) is parallel or substantially parallel to a longitudinal central axis (II-II) of the second stent (120). The membrane-covered stent (100) can prevent or delay a bottom surface (140a) of the recess (140) from being attached to an opening of a branch vessel (101).

## Description

### Technical Field

The present invention relates to the field of interventional medical devices, and more particularly relates to a membrane-covered stent.

### Background Art

This section provides only background information related to the present invention, which is not necessarily the existing art.

Aneurysm and arterial dissection are common vascular diseases clinically. Without medical intervention, aneurysms have the risk of rupture, posing a great threat to the lives of patients.

With the continuous development of medical technology, minimally invasive surgery is used to implant a membrane-covered stent into the body to treat the aneurysms and arterial dissections. Because of its small trauma and rapid recovery, the minimally invasive surgery has been widely used. This therapy is to compress a membrane-covered stent into a delivery device, guide the delivery device into the body of a patient along a pre-implanted guide wire track, release, after the delivery device reaches a diseased position, the membrane-covered stent to isolate the lesion, and rebuild a blood flow channel. After the aneurysm and arterial dissection lose the blood supply, the residual blood in an aneurysm lumen is gradually thrombosed and muscularized into a vascular tissue, and an expanded aneurysm wall shrinks due to a pressure and gradually returns to an original state, so as to achieve the objective of treating the aneurysm and arterial dissection.

When the location of the aneurysm or arterial dissection is close to a branch vessel, the opening of the branch vessel may be blocked after the implantation of the membrane-covered stent, thus causing the blood flow of the branch vessel to be blocked. One of the methods to solve this problem in the prior art is to dispose a recess structure on the membrane-covered stent. After the implantation, the recess structure corresponds to a branch vessel. The recess structure is provided with a window to allow a blood flow to pass and ensure the blood flow supply of the branch vessel. However, after the existing membrane-covered stent with the recess structure is implanted into a blood vessel, the artery at a branch portion will press the recess structure, causing the volume of the recess structure to become smaller. A covering membrane (i.e. a bottom surface of a recess) at the recess structure is fitted to the opening of the branch vessel, causing the blood flow of the branch vessel to be blocked, so that the recess structure cannot play its role. Or, due to the pressure, the window on the recess structure is too close to the opening of the branch vessel, which is not conducive to a subsequent operation of rebuilding the blood supply of the branch vessel, such as an operation of introducing a guide wire into the window during the building of a delivery track, and an operation of introducing a branch stent deliverer into the window.

### Summary of the Invention

Based on this, it is necessary to provide a membrane-covered stent capable of avoiding or slowing down the fitness of a bottom surface of a recess to an opening of a branch vessel.

A membrane-covered stent includes a first stent, a second stent and a covering membrane, wherein the covering membrane is covered on the first stent to form a lumen structure provided with a recess in the middle portion; the second stent is at least partially accommodated in the recess; and a longitudinal central axis of the first stent is parallel or substantially parallel to a longitudinal central axis of the second stent.

In one embodiment, the first stent includes a plurality of first waveform rings and at least one second waveform ring; the plurality of first waveform rings are located at two ends of the first stent; the second waveform ring is located in middle portions of the first stents; and the second waveform ring is radially opposite to the recess.

In one embodiment, the second waveform ring is of a closed-loop structure with wave crests and wave troughs or an open-loop structure with wave crests and wave troughs.

In one embodiment, when the second waveform ring is of the closed-loop structure with wave crests and wave troughs, the second waveform ring includes a first non-closed waveform ring and a first closed portion; the first non-closed waveform ring is an open-loop waveform ring with wave crests and wave troughs; the open-loop waveform ring is provided with two free ends; the first closed portion includes a straight rod; two ends of the straight rod are respectively directly or indirectly connected with the two free ends;
when the second waveform loop is of the open-loop structure with wave crests and wave troughs, the second waveform loop is an open-loop waveform ring with wave crests and wave troughs; the open-loop waveform ring is provided with two free ends; and the two free ends are of passivated structures.

In one embodiment, the second stent includes at least one third waveform ring; and the third waveform ring is of a closed-loop structure with wave crests and wave troughs or an open-loop structure with wave crests and wave troughs.

In one embodiment, a plurality of third waveform rings are provided; and the plurality of third waveform rings are axially arranged at intervals or are axially arranged in a non-spaced manner.

In one embodiment, when each third waveform ring is of the closed-loop structure with wave crests and wave troughs, the third waveform ring includes a second non-closed waveform ring and a second closed portion; the second non-closed waveform ring is an open-loop waveform ring with wave crests and wave troughs; the open-loop waveform ring is provided with two free ends; the second closed portion includes a straight connecting rod; two ends of the straight connecting rod are respectively directly or indirectly connected with the two free ends;
when the third waveform loop is of the open-loop structure with wave crests and wave troughs, the third waveform loop is an open-loop waveform ring with wave crests and wave troughs; the open-loop waveform ring is provided with two free ends; and the two free ends are of passivated structures.

In one embodiment, the recess includes a bottom surface and a side surface surrounding the bottom surface; each third waveform loop is at least partially accommodated in the recess; and the third waveform loop is not fixedly connected with the bottom surface.

In one embodiment, the second stent is an integrated stent formed by braiding braided wires or an integrated stent formed by cutting.

In one embodiment, a first window is formed in one side of the second stent.

In one embodiment, the recess includes a bottom surface and a side surface surrounding the bottom surface; a first window is formed in one side of the second stent close to the bottom surface; the second stent is further provided with a second window; and the first window and the second window are radially opposite.

In one embodiment, the recess includes a bottom surface and a side surface surrounding the bottom surface; and at least one of the bottom surface and the side surface is provided with a through hole.

In one embodiment, a radial supporting strength of the first stent is P1, and a radial supporting strength of the second stent is P2; the P1 and the P2 satisfy: 1/2<(P2/P1)<1; or the P1 and the P2 satisfy: 1≦(P2/P1)≦2.

The second stent of the above membrane-covered stent is at least partially accommodated in the recess. When the membrane-covered stent bends in response to the bending of a blood vessel, the trend of the bottom surface of the recess getting close to a branch vessel is blocked or restrained due to the radial pressing action of the second stent, so that the fitness of the bottom surface of the recess to an opening of the branch vessel can be avoided or slowed down.

### Brief Description of the Drawings

Fig. 1 is a schematic structural diagram of a membrane-covered stent of one embodiment;
Fig. 2 is an equiaxial test diagram of the membrane-covered stent shown in Fig. 1;
Fig. 3 is a side view of a second waveform loop of one embodiment;
Fig. 4 is a front view of a second waveform loop of another embodiment;
Fig. 5 is a side view of the second waveform loop shown in Fig. 4;
Fig. 6 is a top view of the second waveform loop shown in Fig. 4;
Fig. 7 is a front view of a second waveform loop of another embodiment;
Fig. 8 is a side view of the second waveform loop shown in Fig. 7;
Fig. 9 is a top view of the second waveform loop shown in Fig. 7;
Fig. 10 is a front view of a third waveform loop of one embodiment;
Fig. 11 is a top view of the third waveform loop shown in Fig. 10;
Fig. 12 is a side view of the third waveform loop shown in Fig. 10;
Fig. 13 is a front view of a third waveform loop of another embodiment;
Fig. 14 is a side view of a membrane-covered stent of one embodiment;
Fig. 15 is a schematic structural diagram of a second stent of one embodiment;
Fig. 16 is a schematic structural diagram of a second stent of another embodiment;
Fig. 17 is a schematic structural diagram of a membrane-covered stent of another embodiment;
Fig. 18 is a bottom view of a third waveform loop of one embodiment;
Fig. 19 is a bottom view of a third waveform loop of one embodiment;
Fig. 20 is an equiaxial test diagram of a membrane-covered stent of another embodiment;
Fig. 21 is an equiaxial test diagram of a membrane-covered stent of another embodiment;
Fig. 22 is an equiaxial test diagram of a membrane-covered stent of another embodiment;
Fig. 23 is a schematic diagram of a state of implanting the membrane-covered stent shown in Fig. 1 in a blood vessel; and
Fig. 24 is a schematic diagram of a state of implanting a branch stent in the state shown in Fig. 23.

### Detailed Description of the Invention

In order to make the foregoing objectives, features and advantages of the present invention more obvious and understandable, the specific implementation modes of the present invention are described in detail with reference to the accompanying drawings. Many specific details are described in the following descriptions to facilitate full understanding of the present invention. However, the present invention can be implemented in a variety of other ways than those described herein, and those skilled in the art can make similar improvements without departing from the connotation of the present invention. Therefore, the present invention is not limited by specific implementations disclosed below.

Unless otherwise defined, all technical and scientific terms used herein are the same as meanings of general understandings of those skilled in the art of the present invention. The terms used in the description of the present invention herein are merely to describe the specific embodiments, not intended to limit the present invention.

In this text, "proximal end" is defined to be close to the heart, and "distal end" is defined to be away from the heart. "Axial direction" refers to a direction parallel to a connecting line between a center of a distal end of a medical apparatus and a center of a proximal end, and "radial direction" refers to a direction perpendicular to the above axial direction.

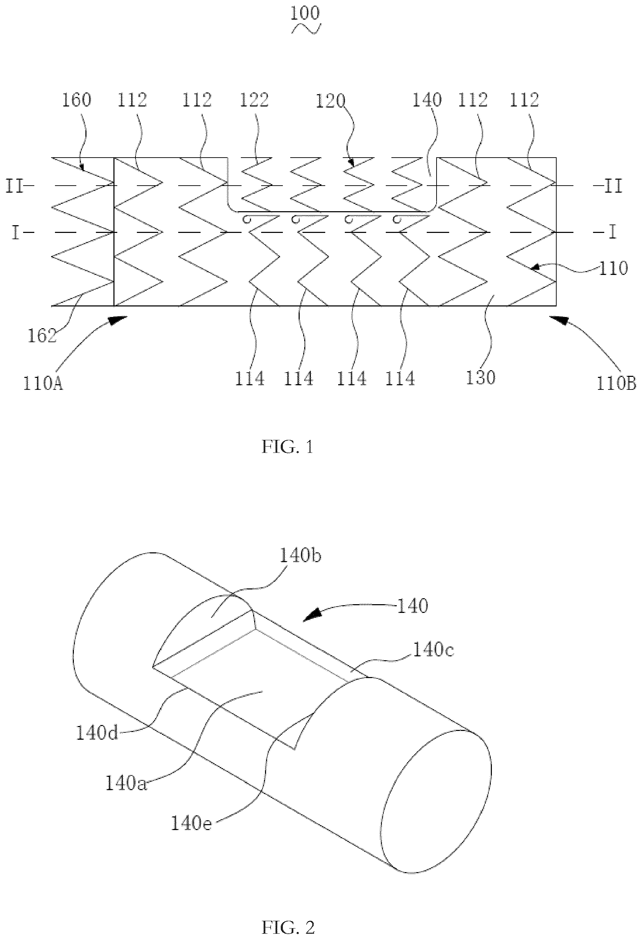

Referring to Fig. 1, a membrane-covered stent 100 of one embodiment includes a first stent 110, a second stent 120 and a covering membrane 130. The covering membrane 130 is covered on the first stent 110 to form a lumen structure with openings at both ends and a recess 140 in a middle portion, as shown in Fig. 2. The lumen structure is provided with an inner cavity communicated with two open ends to allow blood flow to pass. The recess 140 has a bottom surface 140a and a side surface surrounding the bottom surface 140a. In one embodiment, the side face includes a side surface 140b, a side surface 140c, a side surface 140d and a side surface 140e. The side surface 140b and the side surface 140e are axially opposite, and the side surface 140c and the side surface 140d are radially opposite. In the state of no load, the bottom surface 140a is a plane or a curved surface. The curved surface can be a concave surface downwards recessed with respect to an opening of the recess 140, a convex surface upwards bulged with respect to the opening of the recess 140, or a curved surface with both a concave portion and a convex portion. The second stent 120 is at least partially accommodated in the recess 140, and a longitudinal central axis I-I of the first stent 110 is parallel or substantially parallel to a longitudinal central axis II-II of the second stent 120. Being substantially parallel means that an angle between the longitudinal central axis I-I and the longitudinal central axis II-II is greater than 0° and less than 10°.

The first stent 110 and the second stent 120 are made of materials with good biocompatibility and good elasticity, such as nickel-titanium alloy and stainless steel. The covering membrane 130 is made of a highly biocompatible film material, such as PET and PTFE. The way for combining or fixing the covering membrane 130 and the first stent 110 is not limited, for example, high temperature pressurization or suture fixation can also be used. The covering membrane 130 can be of a single-layer structure or a multilayer structure.

In one embodiment, the second stent 120 is completely accommodated in the recess 140. Furthermore, an outer surface of the second stent 120 far away from the bottom surface 140a of the recess 140 is flush with an outer surface of the first stent 110. As shown in Fig. 1, that is, the second stent 120 and the first stent 110 are coplanar in a section of the recess 140 in an opening direction (the section is parallel to the longitudinal central axis I-I and the longitudinal central axis II-II). The entire membrane-covered stent 100 has a uniform outer diameter. Alternatively, an outer surface of the second stent 120 far away from the bottom surface 140a of the recess 140 is lower than an open end of the recess 140, that is, an outer diameter of the second stent 120 is less than a depth of the recess 140. In one embodiment, a difference value between the outer diameter of the second stent 120 and the depth of the recess 140 (or a distance between the section of the second stent 120 parallel to the longitudinal central axis II-II and an end surface of the open end of the recess 140) does not exceed 25% of the outer diameter of the second stent 120, so as to avoid such a phenomenon that an extremely deep recess 140 blocks a main body vessel to affect the blood flow of the main body vessel. In another embodiment, the second stent 120 is partially accommodated in the recess 140, that is, the second stent 120 partially protrudes from the recess 140. A distance between the projecting portion and the end surface of the open end of the recess 140 shall not exceed 10% of the outer diameter of the second stent 120, so as to avoid affecting the anchoring of the first stent 110 of the membrane-covered stent 100 and the main body vessel, or avoid the second stent 120 from excessively pressing a vascular wall to damage the vascular wall.

The first stent 110 has a first end 110A and a second end 110B axially opposite to each other. The first stent 110 includes a plurality of first waveform rings 112 and at least one second waveform ring 114. The plurality of first waveform rings 112 are located at the first end 110A and the second end 110B of the first stent 110. The at least one second waveform ring 114 is located between the first waveform rings 112 at both ends, and is radially opposite to the recess 140. That is, the second waveform ring 114 is located in the middle portion of the first stent 110.

In one embodiment, each first waveform ring 112 is of a Z-shaped waveform ring structure. The Z-shaped waveform ring structure is of a closed-loop structure with wave crests and wave troughs and formed by connecting a plurality of first wave rods (not shown in the figure) end to end. At least one first waveform ring 112 is located at the first end 110A, and at least one first waveform ring 112 is located at the second end 110B. In one embodiment, two or more first waveform rings 112 are located at the first end 110A, and two or more first waveform rings 112 are located at the second end 110B. When the number of the first waveform rings 112 at both ends is greater than or equal to 2, two or more first waveform rings 112 at each end are arranged at intervals along an axial direction (an extending direction of the longitudinal central axis I-I). In addition, two or more first waveform rings 112 at each end are connected by axial connectors (not shown in the figure) or the plurality of first waveform rings 112 are connected together by the covering membrane 130 without any connectors. In other embodiments, the number of the first waveform ring 112 located at the first end 110A and the number of the first waveform ring 112 located at the second end 110B can be equal or unequal.

It should be noted that the waveforms, wave heights, wave numbers, etc. of the plurality of waveform rings 112 located on both sides of the second waveform ring 114 can be the same or different. The number of the first waveform ring 112 located on both sides of the second waveform ring 114 may be equal or unequal.

A radial dimension (a distance between two points farthest from each other, the same below, for example, a diameter) of the second waveform ring 114 is less than a radial dimension (for example, a diameter) of the first waveform ring 112, so that when the covering membrane 130 is covered on the first stent 110 to form a lumen structure with two open ends and the recess 140 in the middle portion.

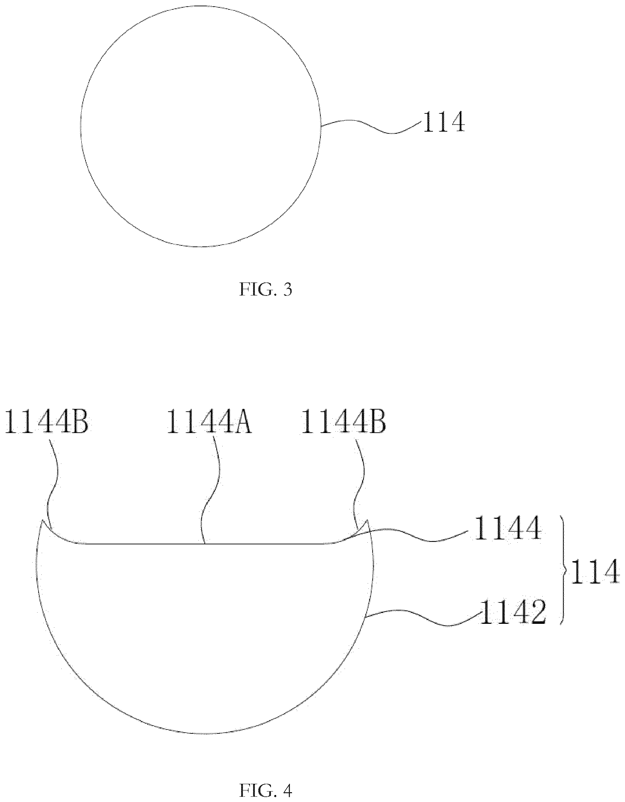

In one embodiment, the second waveform ring 114 is of a closed-loop structure with wave crests and wave troughs and formed by connecting a plurality of second wave rods (not shown in the figure) end to end, as shown in Fig. 3, but the diameter of the second waveform ring 114 is less than that of the first waveform ring 112. The second waveform ring 114 is opposite to the bottom surface 140a of the recess 140, and radially supports a region of the covering membrane 130 located on the bottom surface 140a. The second waveform ring 114 is fixedly connected with the region of the covering membrane 130 located on the bottom surface 140a. Alternatively, the second waveform ring 114 is fixedly connected with at least one of portions of the covering membrane 130 located on the side surface 140b, the side surface 140c, the side surface 140d and the side surface 140e, but is not connected with a portion of the covering membrane 130 located on the bottom surface 140a. The connection way includes but is not limited to direct fixed connection by means of gluing and suturing. Alternatively, the second waveform ring 114 and the covering membrane 130 are indirectly connected by a connector.

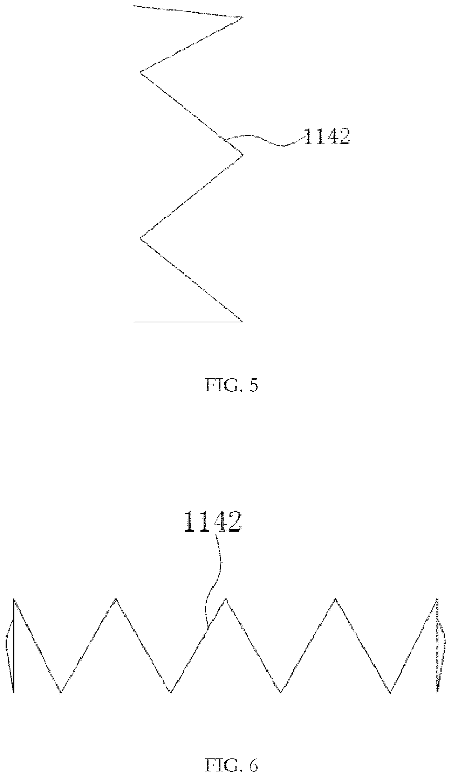

In one embodiment, as shown in Fig. 4, the second waveform ring 114 is of a closed-loop structure, including a first non-closed waveform ring 1142 and a first closed portion 1144. Referring to Fig. 5 and Fig. 6 together, the first non-closed waveform ring 1142 is of a non-closed structure with wave crests and troughs and formed by connecting a plurality of wave rods (not shown in the figure) end to end, with end portions not connected. In one embodiment, when the bottom surface 140a of the recess 140 is a plane, the first non-closed waveform ring 1142 is 1/2 to 3/4 of a circular structure. When the bottom surface 140a of the recess 140 is a concave surface, the first non-closed loop 1142 is 5/9 to 8/9 of a circle. When the bottom surface 140a of the recess 140 is a concave surface, a non-closed waveform ring 1142 is 1/4 to 5/9 of a circle. The two ends of the first closed portion 1144 are respectively connected with the two free ends of the first non-closed loop 1142 to form the closed-loop structure. The first closed portion 1144 is of a rod piece structure, including a straight rod 1144A. The straight rod 1144A is opposite to the bottom surface 140a. The straight rod 1144A of the second waveform ring 114 of this structure has a planar region, which can be well matched with the bottom surface 140a of the recess 140, so as to better radially support the region of the covering member 130 located on the bottom surface 140a and facilitate maintaining the bottom surface 140a of the recess 140 as a plane or substantially as a plane, thus helping to maintain the shape of the recess 140, and avoiding such a phenomenon that the blood flow of the branch vessel is blocked since the recess 140 deforms to make the region of the covering member 130 located on the recess 140 fitted to the opening of the branch vessel.

The second waveform ring 114 is fixedly connected, through the straight rod 1144A, with the region of the covering membrane 130 located on the bottom surface 140a. The second waveform ring can also be fixedly connected with an inner surface of the covering membrane 130 through other portions, while the straight rod 1144A is not fixedly connected with the region of the covering membrane 130 located on the bottom surface 140a, that is, the recess 140 only overlaps the straight rod 1144A of the second waveform ring 114, and is not fixed by gluing, suturing and the like.

In one embodiment, the first closed portion 1144 also includes two arc-shaped rods 1144B which are respectively connected with the two ends of the straight rod 1144A, and ends of the two arc-shaped rods 1144B far away from the straight rod 1144A are respectively connected with the two free ends of the first non-closed loop 1142. The arc-shaped rods 1144B are arranged to form a transition and avoid stress concentration, so as to avoid the risk of fracture of a connection portion between the first closed portion 1144 and the first non-closed waveform ring 1142.

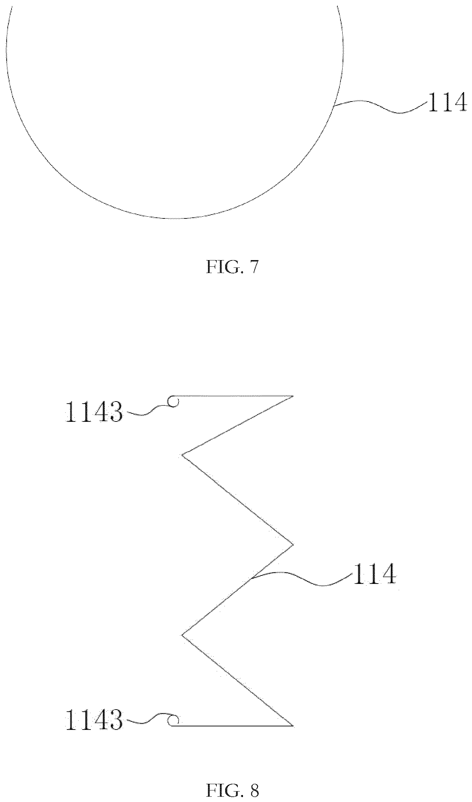

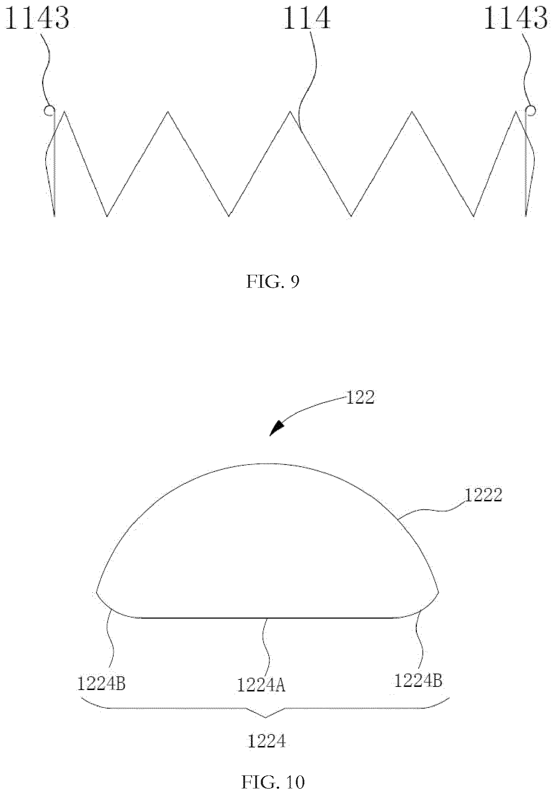

In one embodiment, the first closed portion 1144 is omitted, that is, the second waveform ring 114 only includes the first non-closed waveform ring 1142, so that the second waveform ring 114 is of a non-closed loop structure, as shown in Fig. 7. The two free ends of the second waveform ring 114 are of a passivated structure to avoid puncturing the covering membrane 130. In one embodiment, as shown in Fig. 8 and Fig. 9, the two free ends of the second waveform ring 114 curl respectively in a inwards direction or an outwards direction to form two passivated structures 1143. In another embodiment, the passivated structure can be in other ways, such as a spherical structure fixed to the free ends of the second waveform ring 114 by welding or in other fixing manners.

Referring back to Fig. 1, in one embodiment, the second stent 120 includes at least one third waveform ring 122. In one embodiment, there are a plurality of third waveform rings 122, and the plurality of the third waveform rings 122 are arranged in the recess 140 at intervals along the longitudinal central axis II-II. In another embodiment, the plurality of third waveform rings 122 are arranged in the recess 140 in a non-spaced manner along the longitudinal central axis II-II.

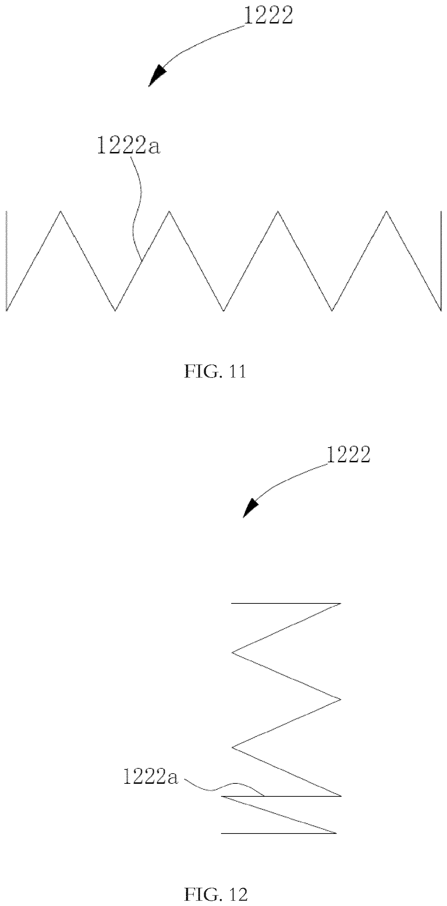

Referring to Fig. 10, in one embodiment, the third waveform ring 122 is of a closed loop structure. The third waveform ring 122 includes a second non-closed waveform ring 1222 and a second closed portion 1224. Referring to Fig. 11, the second non-closed waveform ring 1222 is of a non-closed loop structure formed by connecting a plurality of wave rods 1222a end to end, with end portions not connected, so that the second non-closed waveform ring 1222 has two free ends. In one embodiment, when the bottom surface 140a of the recess 140 is a plane, the second non-closed waveform ring 1222 is 1/2 to 3/4 of a circular structure. When the bottom surface 140a of the recess 140 is a concave surface, the first non-closed loop 1142 is 5/9 to 8/9 of a circle. When the bottom surface 140a of the recess 140 is a concave surface, a non-closed waveform ring 1142 is 1/4 to 5/9 of a circle. The second closed portion 1224 includes a straight connecting rod 1224A and two arc-shaped transitional rods 1224B respectively connected with two ends of the straight connecting rod 1224A. Two ends of the two arc-shaped transitional rods 1224B are respectively connected with the two free ends of the second non-closed waveform ring 1222, thus forming the closed-loop third waveform ring 122.

The third waveform ring 122 is accommodated in the recess 140, and the second closed portion 1224 of the third waveform ring 122 is arranged on the bottom surface 140a of the recess 140. More specifically, the straight connecting rod 1224A of the second closed portion 1224 is arranged on the bottom surface 140a of the recess 140. The radian of the second non-closed waveform ring 1222 matches the radian of the first waveform ring 112 of the first stent 110, so that the outline of the membrane-covered stent 100 is a hollow or substantially hollow cylindrical structure.

Therefore, since the third waveform ring 122 includes the second non-closed waveform ring 1222 and the second closed portion 1224, the third waveform ring can not only match the shape of the recess 140, but also match the radian of the first waveform ring 112, so as to provide a better radial support for the recess 140, and enable the external outline of the membrane-covered stent 100 to be smoothly transitioned, so that the outline of the membrane-covered stent 100 is of a hollow or substantially hollow cylindrical structure to facilitate delivery and release.

In one embodiment, the third waveform ring 122 is fixed on the side surface 140d through the wave rods 1222a, so that the third waveform ring 122 is fixed in the recess 140. In one embodiment, as shown in Fig. 12, when the third waveform ring 122 is located in the recess 140, one wave rod 1222a of the third waveform ring 122 is fixedly connected with an edge line of the side surface 140d, and the other wave rod 1222a of the third waveform ring 112 is fixedly connected with an edge line of the side surface 140c.

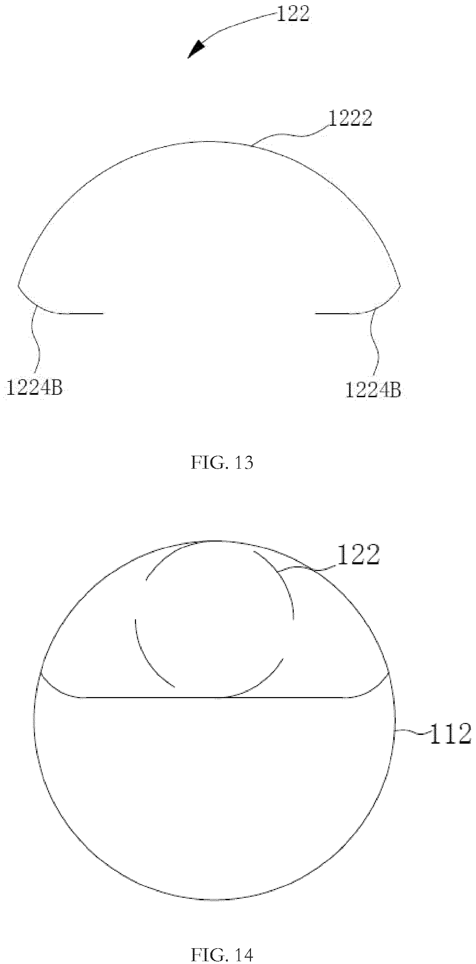

In one embodiment, referring to Fig. 13, for the structure of the third waveform ring 122, the straight connecting rod 1224A is omitted, that is, the third waveform ring 122 includes a second non-closed waveform ring 1222 and two arc-shaped transitional rods 1224B respectively connected with two free ends of the second non-closed waveform ring 1222, so that the third waveform ring 122 has an opening and is of a non-closed loop structure. The wave rods 1222a of the third waveform ring 122 are fixedly connected with the edge lines of the side surfaces 140d and 140c to fix the third waveform ring 122 in the recess 140. Alternatively, the wave rods 1222a of the third waveform ring 122 is connected with the side surface 140d and the side surface 140c to fix the third waveform ring 122 in the recess 140, but the connection portion is not necessarily located on the edge lines of the side surface 140d and the side surface 140c.

Omitting the straight connecting rod 1224A is conducive to reducing the consumption of metals. In addition, under a radial force facing the bottom surface 140a of the recess 140, the second non-closed waveform ring 1222 of the third waveform ring 122 can press the bottom surface 140a of the recess 140 to prevent the bottom surface 140 of the recess 140d from being fitted to the opening of the branch vessel.

A free end of each arc-shaped transitional rod 1224B is of a passivated structure to avoid damaging the covering membrane 130. The form of the passivated structure is not limited. For example, the passivated structure can be the same as the passivated structure of the second waveform ring 114. It will not be repeated here.

In another embodiment, referring to Fig. 14, the third waveform ring 122 is of a closed-loop structure with wave crests and wave troughs and formed by connecting a plurality of wave rods end to end, that is, the third waveform ring 112 is a cylindrical waveform ring. a contact area between the cylindrical third waveform ring 122 and the bottom surface 140a of the recess 140 is small, which is conducive to the subsequent windowing operation.

In one implementation, the cylindrical third waveform ring 122 is fixedly connected with the side surface 140c and the side surface 140d of the recess 140 by means of the wave rods which are radially opposite, and a bottom portion of the third waveform ring 122 is not connected with the bottom surface 140a of the recess 140.

It should be noted that when there are a plurality of third waveform rings 122, the plurality of third waveform rings 122 are arranged along the axial direction (the extending direction of the longitudinal central axis II-II) at intervals. Adjacent third waveform rings 122 can be connected through an axial connector (not shown in the figure), and may not be connected. Arranging the axial connector can stabilize the release position of the third waveform ring 122.

When the adjacent third waveform rings 122 are connected by the axial connector, the axial connector can be a rigid connector or a flexible connector.

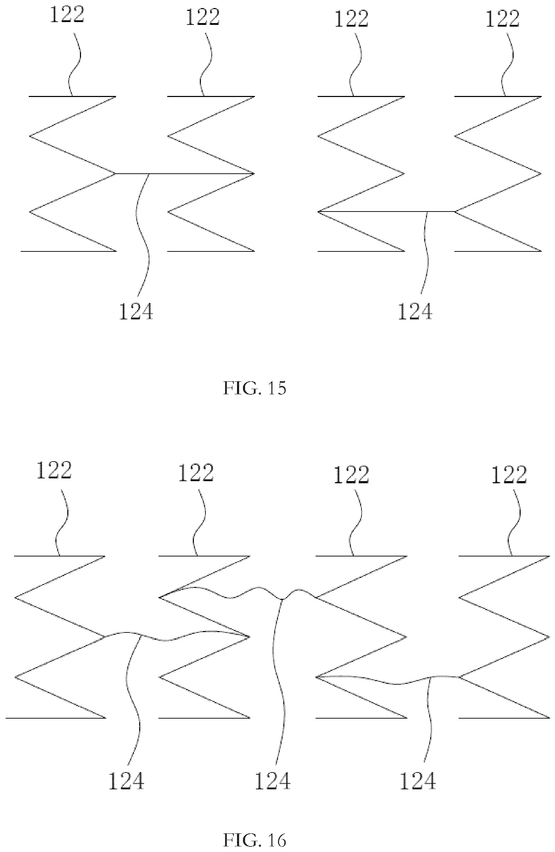

The form of the rigid connector is not limited. It can be a straight rod, a specially-shaped rod, or the like made of a metal material. Referring to Fig. 15, in one embodiment, the third waveform rings 122 can be connected by an axial connector 124. The axial connector 124 is a straight rodlike rigid connector. Furthermore, when the membrane-covered stent 100 is bent, a large curvature side and a small curvature side are formed. The axial connector 124 is located at the large curvature side, so as not to affect the bending of the membrane-covered stent 100. In one embodiment, the number of the axial connector 124 does not exceed two. In one embodiment, the number of the axial connector 124 is 1, that is, only one axial connector 124 is used to connect two adjacent third waveform rings 122.

Referring to Fig. 16, in one embodiment, the axial connector 124 is a flexible connector. The position and number of the flexible connector are not limited. In this embodiment, the number of the flexible connector is three, and the three flexible connectors are located on one side of the second stent 120 far away from the bottom surface 140a of the recess 140.

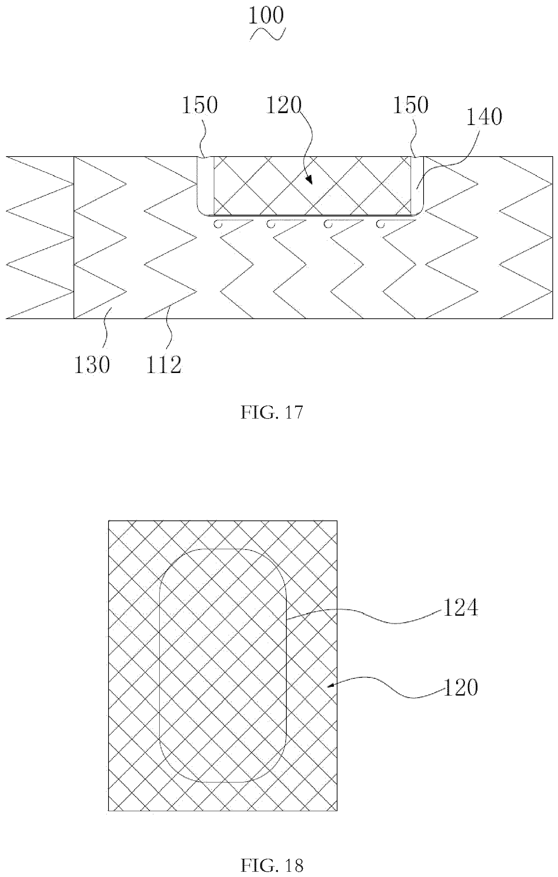

In another embodiment, referring to Fig. 17, the second stent 120 is an integrated metal skeleton. For example, the second stent 120 is a lumen net structure formed by braiding metal wires or cutting a metal tube. Alternatively, the second stent 120 includes a plurality of third waveform rings 122 (not shown in Fig. 17) arranged in a non-spaced manner, and the wave crests and wave troughs of two adjacent third waveform rings 122 are axially opposite and connected, so that the wave crests and wave troughs of each third waveform ring 122 are no longer in a state with free ends. The second stent 120 of this structure is of an integrated structure, which can avoid such a phenomenon that the free ends of the wave crest and/or wave trough of a single waveform ring can be easily pushed into the branch vessel, thus avoiding the damage to the branch vessels caused by the single waveform ring, and improving the safety of use.

In one embodiment, the bottom portion of the second stent 120 is connected with the bottom surface 140a of the recess 140. In another embodiment, side surfaces of the second stent 120 are connected with the side surface 140c and the side surface 140d of the recess 140.

In another embodiment, as shown in Fig. 17, the second stent 120 is connected with the covering membrane 130 or the first waveform ring 112 through an axial connecting rod 150. One end of the axial connecting rod 150 is connected with a first end of the second stent 120, and the other end extends axially out of the recess 140 and is connected with the covering membrane 130 or the first waveform ring 112.

There shall be at least two axial connecting rods 150. When there are two axial connecting rods 150, the two axial connecting rods 150 are located at the two axially opposite ends of the second stent 120, and the two axial connecting rods 150 are both located at the open ends of the recess 140. One end of each axial connecting rod 150 is connected with the second stent 120, and the other end extends axially from the open end of the recess 140 to the outside of the recess 140a, and is connected with the covering membrane 130 or the first waveform ring 112.

In addition, the bottom portion (a portion close to the bottom surface 140a of the recess 140) of the second stent 120 is not fixedly connected with the bottom surface 140a of the recess 140, so as to facilitate the adjustment of the relative position between the bottom portion of the second stent 120 and the bottom surface 140a of the recess 140, and facilitate the subsequent windowing and branch stent implanting operations.

The form of the axial connecting rod 150 is not limited. For example, it can be a straight rod, a specially-shaped rod, or the like.

It can be understood that in the embodiment where the second stent 120 is the integrated metal skeleton, the shape of a cross section of the second stent 120 is not limited. For example, the second stent can be of the structure shown in Fig. 10 that includes the second non-closed waveform ring 1222, the straight connecting rod 1224A and the arc-shaped transitional rods 1224B, or can be of the structure shown in Fig. 13 that includes the second non-closed waveform ring 1222 and the arc-shaped transitional rods 1224B, but the straight connecting rod 1224A is omitted. The second stent can also be of a cylindrical structure as shown in Fig. 14, which will not be described repeatedly in detail here.

Referring to Fig. 18, in another embodiment, a first window 124 is formed in the bottom portion (the portion close to the bottom surface 140a of the recess 140) of the second stent 120. The first window 124 is located in a middle region of the second stent 120. Since the window 124 is not blocked or interfered by the metal skeleton partially, the subsequent windowing and branch stent implanting operations are easier. In addition, it is beneficial to reduce the use of metals. At the same time, since the radial dimension of the compressed second stent 120 is reduced, the radial dimension of the compressed membrane-covered stent 100 is reduced, so that a smaller delivery sheath can be used for delivery, causing less injury to patients or being applicable for more patients.

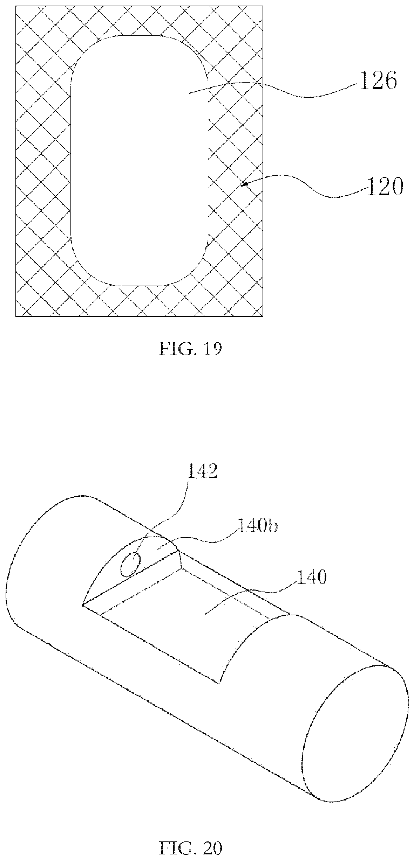

Referring to Fig. 19, in another embodiment, a second window 126 is also arranged at a portion of the second stent 120 radially opposite to the first window 124, and the first window 124 is opposite to the second window 126, that is, the middle region of the second stent 120 is not provided with the metal skeleton. In this way, the metal skeleton at the edges of the second window 124 and the second window 126 is used for supporting, so that after the membrane-covered stent 100 is implanted into a blood vessel, the metal skeleton presses the bottom surface 140a of the recess 140 to prevent the bottom surface 140a of the recess 140 from moving towards the opening of a branch vessel and being fitted to the opening of the branch vessel. In addition, while ensuring the supporting effect, it is more convenient for the subsequent windowing and branch stent implanting operations. In addition, the dosage of metal is smaller, and the radial dimension of the compressed membrane-covered stent 100 is smaller, so that a smaller delivery sheath can be used for delivery, causing less injury to patients, or being applicable to more patients.

In another embodiment, when the second window 126 is omitted, the first window 124 is formed in the bottom portion of the second stent 120, or the first window 124 can be formed in a side (i.e. a top portion of the second stent 120) of the second stent 120 opposite to the bottom portion.

In one embodiment, the recess 140 is provided with a through hole running through a side surface of the recess 140 to allow the blood flow to pass. In one embodiment, at least one of the bottom surface 140a, the side surface 140b, the side surface 140c, the side surface 140d and the side surface 140e of the recess 140 is provided with a through hole. Specifically, referring to Fig. 20, in one embodiment, the recess 140 is provided with a first through hole 142 running through the side surface 140b. The blood flow can flow from the main body vessel to each branch vessel through the first through hole 142. When the lesion does not involve the root of the branch artery, there is no need to implant a branch stent later, which is more economical for patients.

When the first window 124 is formed in the top portion of the second stent 120, the first through hole 142 and the first window 124 can be used as a channel for aa branch stent. The branch stent enters the second stent 120 from the first through hole 142, and extends out of the second stent 120 from the first window 124.

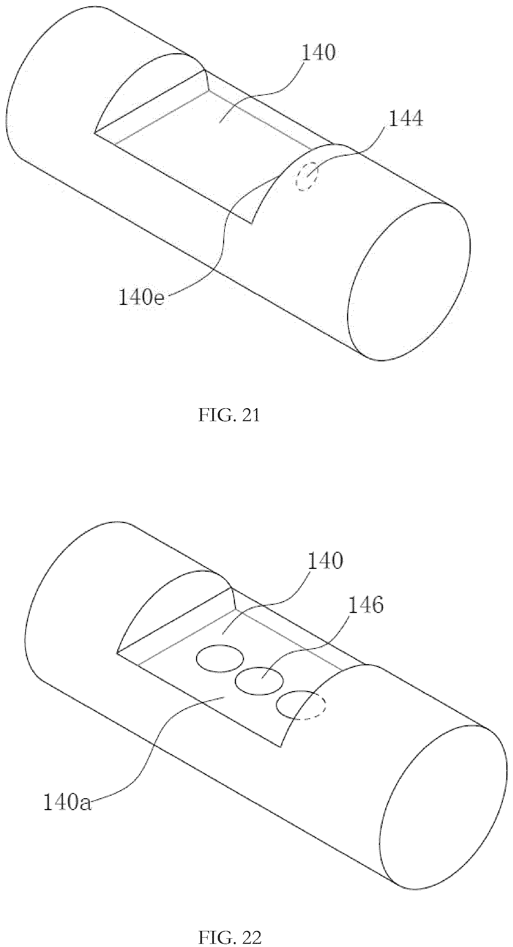

Referring to Fig. 21, in one embodiment, the recess 140 is provided with a second through hole 144 running through the side surface 140e, which can also realize that the blood flow flows from the main body vessel to each branch vessel through the second through hole 144. When the lesion does not involve the root of the branch artery, there is no need to implant a branch stent later.

It should be noted that the first through hole 142 and the second through hole 144 can exist at the same time, or either the first through hole 142 or the second through hole 144 can exist.

Referring to Fig. 22, in one embodiment, the recess 140 is provided with a third through hole 146 running through the bottom portion 140a, which can also realize that the blood flow can flow from the main body vessel to each branch vessel through the third through hole 146. When the lesion does not involve the root of the branch artery, there is no need to implant a branch stent later. Or, when there is the third through hole 146, it is not necessary to perform an in situ windowing operation on the bottom portion 140a during the subsequent implantation of the branch stent, but the branch stent is delivered directly through the third through hole 146.

It should be noted that when there is the third through hole 146, at least one of the first through hole 142 and the second through hole 144 can be omitted, or both can be maintained.

It should also be noted that the shapes and numbers of the first through hole 142, the second through hole 144 and the third through hole 146 are not limited, as long as the blood flow can pass and will not adversely affect the overall structure of the recess 140.

In one embodiment, the first through hole 142, the second through hole 144 and the third through hole 146 are all round holes. In addition, there is one first through hole 142, one second through hole 144, and three third through holes 146.

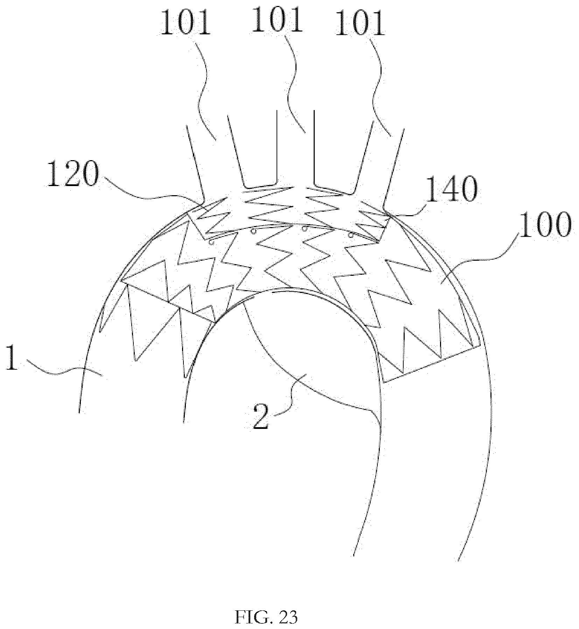

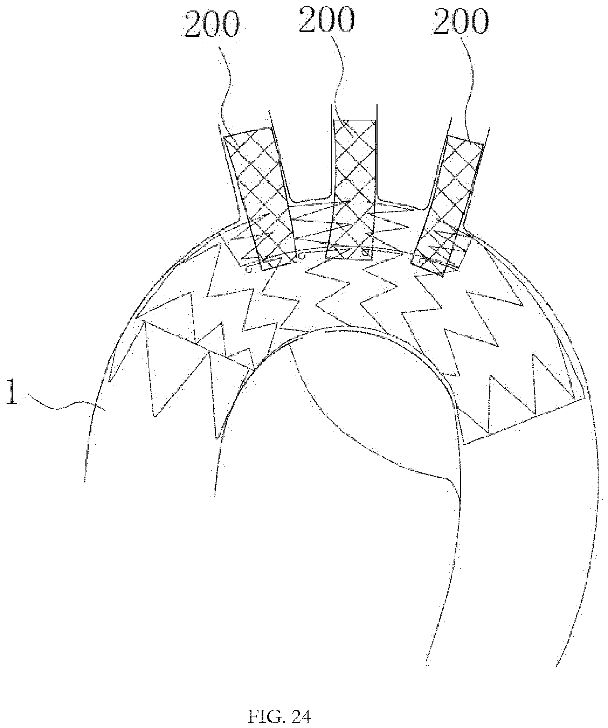

A use method of the membrane-covered stent 100 is described below with the embodiment shown in Fig. 1. As shown in Fig. 23, a diseased vessel 1 is the aortic arch vessel, and one side of the arch opposite to a branch vessel has an arterial dissection 2. After the membrane-covered stent 100 is implanted into the diseased vessel 1, the recess 140 corresponds to the branch vessel. Specifically, three branch vessels 101 are radially opposite to the recess 140. After the membrane-covered stent 100 is implanted into the diseased vessel 1, the membrane-covered stent 100 bends in response to the bending of the diseased vessel 1 itself. In the bending process, a portion where the recess 140 is located bends together, causing the bottom surface 140a (not shown in Fig. 23) of the recess 140 to have a trend of protruding toward the branch vessels 101. However, due to the radial supporting performance, the second stent 120 can provide a reverse supporting force for the bottom surface 140a of the recess 140 to restrain the trend of the bottom surface 140a of the recess 140 for protruding towards the branch vessels 101, so that the bottom surface 140a of the recess 140 can be far away from the openings of the branch vessels 101, so as to prevent the covering membrane 130 from blocking the openings of the branch vessels 101. Referring to Fig. 24 together, next, an in situ windowing technology is used to form a window running through the bottom surface 140a (not shown in Fig. 23) of the recess 140. The branch stent 200 is delivered into the branch vessel 101 from the window and is released to establish a blood flow channel between the branch vessel 101 and the diseased vessel 1.

Due to the radial supporting effect of the second stent 120, the shape of the recess 140 can be well maintained, so that the bottom surface 140a of the recess 140 can keep a sufficient distance from the opening of the branch vessel 101, so as to facilitate in situ windowing and implantation of the branch stent 200.

Moreover, since the bottom portion of the second stent 120 is not fixedly connected with the bottom surface 140a of the recess 140, in the processes of in situ windowing and implantation of the branch sent 200, the position of the third waveform ring 122 can be adjusted according to a requirement, so that the operations are more convenient, which is conductive to improving the position accuracy and shortening the operation time. At the same time, after the branch stent 200 is released completely, the third waveform ring 122 is fixed around the branch stent 200 to form a stable support, maintain the stability of the position of the branch stent 200, and keep the branch vessels continuously unblocked.

It should be noted that the second waveform rings 114 and the third waveform rings 122 of different embodiments are respectively described above. The second waveform rings 114 and the third waveform rings 122 of different embodiments can be combined arbitrarily.

For example, the second waveform ring 114 shown in Fig. 7 can be combined with the third waveform ring 122 shown in Fig. 10. The straight connecting rod 1224A of the third waveform ring well matches with the bottom surface 140a of the recess 140, which can better press the bottom surface 140a of the recess 140 and prevent the bottom surface 140a from being fitted to the openings of the branch vessels. In addition, the second waveform ring 114 is of an open structure, which is conducive to reducing the dosage of metal and reducing the radial dimension of the compressed membrane-covered stent 100.

For another example, the second waveform ring 114 shown in Fig. 4 can be combined with the third waveform ring 122 shown in Fig. 10, so that the straight rod 1144A of the second waveform ring 114 and the straight connecting rod 1224A of the third waveform ring 122 are opposite and cooperate with each other.

For another example, the second waveform ring 114 shown in Fig. 7 can be combined with the third waveform ring 122 shown in Fig. 13, and the third waveform ring 122 applies a radial supporting force to the recess 140 through the second non-closed waveform ring 1222. This combination uses less metal.

For another example, the second waveform ring 114 shown in Fig. 7 can be combined with the third waveform ring 122 shown in Fig. 14, and the third waveform ring 122 applies a radial supporting force to the recess 140 through the second non-closed waveform ring 1222.

For another example, the second waveform ring 114 shown in Fig. 7 can be combined with the second stent 120 shown in Fig. 17 to provide a radial supporting force for the recess 140 through the second stent 120. In addition, the second stent 120 of this structure can avoid the risk of damaging an inner wall of a branch vessel by the third waveform ring 122 with the free ends at the end portions.

For another example, the second waveform ring 114 shown in Fig. 7 can be combined with the third waveform ring 122 shown in Fig. 18 or Fig. 19.

For another example, the second waveform ring 114 shown in Embodiment 4 can be combined with the third waveform ring 122 shown in Fig. 13. The straight rod 1144A of the second waveform ring 114 presses the bottom surface 140a of the recess 140, and the dosage of metal is reduced, that is, the radial dimension of the compressed membrane-covered stent 100 is reduced.

Other combination methods will not be described one by one.

No matter how the second waveform ring 114 and the third waveform ring 122 of different structures are combined, in one embodiment, the radial supporting strength P1 of the first stent 110 and the radial supporting strength P2 of the second stent 120 are different.

In one embodiment, P1 and P2 satisfy: 1/2<(P2/P1)<1, that is, the radial supporting strength P2 of the second stent 120 is less than the radial supporting strength P1 of the first stent 110, but P2 should be high enough, greater than 0.5 times of P1, so that when the first stent 110 and the second stent 120 are subjected to the same radial compression force, a decrease of the radial dimension of the second stent 120 after the radial compression is not too large, so as to facilitate the windowing operation.

In one embodiment, P1 and P2 satisfy: 1≦(P2/P1)≦2, that is, the radial supporting strength P2 of the second stent 120 is greater than or equal to the radial supporting strength P1 of the first stent 110, and P2 is less than twice P1, so that when the first stent 110 and the second stent 120 are subjected to the same radial compression force, a decrease of the radial dimension of the first stent 110 after the radial compression is not too large, so as to keep the blood flow through the first stent 110 unblocked.

It should be noted that a radial supporting strength is equal to a ratio of a radial supporting force to an axial length. For example, when the radial supporting force on the second stent 120 is F2, and the axial length of the second stent 120 is L2, P2=F2/L2. When the radial supporting force on the first stent 110 is F1, and the axial length of the first stent 110 is L1, P1=F1/L1. L1 is the axial length of a portion of the first stent 110 radially opposite to the second stent 120, L1=L2.

It should also be noted that a plate compression method can be used to test the radial supporting force F 1 of the first stent 110 and the radial supporting force F2 of the second stent 120. Or, a radial compression method can be used to test the radial supporting force F1 of the first stent 110 and the radial supporting force F2 of the second stent 120. That is, to compare F1 with F2, F1 and F2 are tested using the same test method under the same conditions. For example, the radial compression method is used to test the radial supporting force F1 when the first stent 110 is compressed by 50% and the radial supporting force F2 when the second stent 120 is compressed by 50%. When the plate compression method is adopted, two flat plates compress both sides of a stent respectively. The two flat plates are parallel and are symmetrically arranged about the axis of symmetry of the longitudinal central axis. In addition, the two flat plates are respectively two tangent planes of the stent.

The radial supporting force F1 of the first stent 110 is related to a rod diameter, wave number, included angle at the wave trough or wave crest and other parameters of the second waveform ring 114, and the radial supporting force F2 of the second stent 120 is related to a rod diameter, wave number, included angle at the wave trough or wave crest of the third waveform ring 122, which can be adjusted by those skilled in the art as required.

Referring back to Fig. 1 again, in one embodiment, the membrane-covered stent 100 also includes an anchorage stent 160. The anchorage stent 160 is located at a first end 110A of the first stent 110 and is connected to the first waveform ring 112 or the covering membrane 130. The anchorage stent 160 is a bare stent, excluding any covering membrane. The anchorage stent 160 includes at least one anchorage waveform ring 162. When there are a plurality of anchorage waveform rings 162, the plurality of anchorage waveform rings 162 are arranged at intervals along the longitudinal central axis I-I.

When the membrane-covered stent 100 is implanted into a blood vessel, the anchorage stent 160 is located at the proximal end of the membrane-covered stent 100. The anchorage stent 160 is arranged to further improve the anchorage performance of an end portion of the membrane-covered stent 100.

In one embodiment, one first waveform ring 110 is located at the first end 110A. When the recess 140 is only radially opposite to two branch vessels 101 (two branch vessels 101 close to the second end 110B), one first waveform ring 110 cooperates with the anchorage stent 160 to achieve the anchorage of the proximal end of the membrane-covered stent 100. Moreover, since the anchorage stent 160 is a bare stent, it will not completely cover the opening of the third branch vessel 101, so there is no need to form a window and implant a branch stent 200.

In one embodiment, a length of a wave rod of the anchorage waveform ring 162 is less than a length of the wave rod of the first waveform ring 110, and the wave number of the anchorage waveform ring 162 is greater than the wave number of the first waveform ring 110, so that the anchorage performance of the anchorage stent 160 is better.

The technical features of the embodiments described above can be arbitrarily combined. In order to make the description concise, all possible combinations of various technical features in the above embodiments are not completely described. However, the combinations of these technical features should be considered as the scope described in this specification as long as there is no contradiction in them.

The above-mentioned embodiments only express several implementation modes of the present invention, and their descriptions are more specific and detailed, but they cannot be understood as limiting the patent scope of the present invention. It should be noted that those of ordinary skill in the art can further make various transformations and improvements without departing from the concept of the present invention, and these transformations and improvements all fall within the protection scope of the present invention. Therefore, the protection scope of the patent of the present invention shall be subject to the appended claims.

## Claims

1. A membrane-covered stent, comprising a first stent, a second stent and a covering membrane, wherein the covering membrane is covered on the first stent to form a lumen structure provided with a recess in the middle portion; the second stent is at least partially accommodated in the recess; and a longitudinal central axis of the first stent is parallel or substantially parallel to a longitudinal central axis of the second stent.

2. The membrane-covered stent according to claim 1, wherein the first stent comprises a plurality of first waveform rings and at least one second waveform ring; the plurality of first waveform rings are located at two ends of the first stent; the second waveform ring is located in middle portions of the first stents; and the second waveform ring is radially opposite to the recess.

3. The membrane-covered stent according to claim 2, wherein the second waveform ring is of a closed-loop structure with wave crests and wave troughs or an open-loop structure with wave crests and wave troughs.

4. The membrane-covered stent according to claim 3, wherein when the second waveform ring is of the closed-loop structure with wave crests and wave troughs, the second waveform ring comprises a first non-closed waveform ring and a first closed portion; the first non-closed waveform ring is an open-loop waveform ring with wave crests and wave troughs; the open-loop waveform ring is provided with two free ends; the first closed portion comprises a straight rod; two ends of the straight rod are respectively directly or indirectly connected with the two free ends;
when the second waveform loop is of the open-loop structure with wave crests and wave troughs, the second waveform loop is an open-loop waveform ring with wave crests and wave troughs; the open-loop waveform ring is provided with two free ends; and the two free ends are of passivated structures.

5. The membrane-covered stent according to claim 1, wherein the second stent comprises at least one third waveform ring; and the third waveform ring is of a closed-loop structure with wave crests and wave troughs or an open-loop structure with wave crests and wave troughs.

6. The membrane-covered stent according to claim 5, wherein a plurality of third waveform rings are provided; and the plurality of third waveform rings are axially arranged at intervals or are axially arranged in a non-spaced manner.

7. The membrane-covered stent according to claim 5 or 6, wherein when each third waveform ring is of the closed-loop structure with wave crests and wave troughs, the third waveform ring comprises a second non-closed waveform ring and a second closed portion; the second non-closed waveform ring is an open-loop waveform ring with wave crests and wave troughs; the open-loop waveform ring is provided with two free ends; the second closed portion comprises a straight connecting rod; two ends of the straight connecting rod are respectively directly or indirectly connected with the two free ends;
when the third waveform loop is of the open-loop structure with wave crests and wave troughs, the third waveform loop is an open-loop waveform ring with wave crests and wave troughs; the open-loop waveform ring is provided with two free ends; and the two free ends are of passivated structures.

8. The membrane-covered stent according to claim 5 or 6, wherein the recess comprises a bottom surface and a side surface surrounding the bottom surface; each third waveform loop is at least partially accommodated in the recess; and the third waveform loop is not fixedly connected with the bottom surface.

9. The membrane-covered stent according to claim 1, wherein the second stent is an integrated stent formed by braiding braided wires or an integrated stent formed by cutting.

10. The membrane-covered stent according to claim 9, wherein a first window is formed in one side of the second stent.

11. The membrane-covered stent according to claim 9, wherein the recess comprises a bottom surface and a side surface surrounding the bottom surface; a first window is formed in one side of the second stent close to the bottom surface; the second stent is further provided with a second window; and the first window and the second window are radially opposite.

12. The membrane-covered stent according to claim 1, wherein the recess comprises a bottom surface and a side surface surrounding the bottom surface; and at least one of the bottom surface and the side surface is provided with a through hole.

13. The membrane-covered stent according to claim 1, wherein a radial supporting strength of the first stent is P1, and a radial supporting strength of the second stent is P2; the P1 and the P2 satisfy: 1/2<(P2/P1)<1; or the P1 and the P2 satisfy: 1 ≦(P2/P1) ≦2.
